# EUROPÄISCHE PATENTSCHRIFT

(11) **EP 0 706 834 B1**
(45) Veröffentlichungstag und Bekanntmachung des Hinweises auf die Patenterteilung: **03.11.1999**
(21) Anmeldenummer: 95115015.0
(22) Anmeldetag: 23.09.1995
(51) Int. Cl.: B05D 3/06

(54) **Verfahren zur Herstellung eines mit UV-härtbarem Lack beschichteten flächenförmigen Trägermaterials**
Process for coating a flat substrate with an U.V. curable lacker
Procédé pour revêtir un substrat plat avec une laque réticulable aux U.V.

(30) Priorität: 11.10.1994 DE 4436249; 04.11.1994 DE 4439350
(43) Veröffentlichungstag der Anmeldung: 17.04.1996
(73) Patentinhaber: ALKOR GMBH KUNSTSTOFFE, D-81451 München (DE)
(72) Erfinder: Schneider, Manfred, Dipl. Ing., D-86928 Hagenheim (DE); Ewald, Egon, D-81671 München (DE)
(74) Vertreter: Seiler, Siegfried

(56) Entgegenhaltungen:
- EP-A- 0 440 178
- WO-A-81/00683
- DE-A- 2 355 657
- FR-A- 2 265 804
- US-A- 4 326 001

## Beschreibung

Die vorliegende Erfindung betrifft ein Verfahren zur Herstellung eines mit UV-härtbarem Lack beschichteten flächenförmigen Trägermaterials mit matter oder glänzender Oberfläche, wobei das Trägermaterial aus mindestens einer ein- oder mehrschichtigen Kunststoffolienbahn oder Papierbahn mit einer Gesamtdicke von 80 bis 500 µm besteht und unmittelbar darauf oder über eine darauf befestigte Zwischenschicht oder Kunststoffzwischenschicht eine strahlenhärtbare Lackschicht aufgebracht wird, die mindestens ein monomeres Acrylat (mono-, di-, tri- und/oder tetrameres Acrylat) sowie gegebenenfalls übliche Zusatzstoffe enthält, wobei die strahlenhärtbare Lackschicht aus einem Lack auf der Basis eines Acrylatgemisches besteht, das durch Umsetzung mindestens eines prepolymeren Acrylates mittleren Molekulargewichtes von 500 bis 2000 mit dem monomeren Acrylat (mono-, di-, tri- und/oder tetrameren Acrylat) hergestellt wird, und der Lack je 100 Gew.-Teile des Acrylatlackes (berechnet ohne jedes Zusatzmittel oder Verarbeitungshilfsmittel), 1 bis 10 Gew.-Teile eines UV-Initiators oder UV-Initiatorgemisches enthält oder daraus besteht und daß die aufgebrachte Lackschicht anschließend durch UV-Strahlen ausgehärtet wird. Gemäß der Erfindung wird der Mattgrad des UV-Lackes durch eine bestimmte Auswahl der Monomeren eingestellt, wobei zusätzlich für die UV-Härtung bestimmte Bedingungen eingehalten werden müssen.

Aus der EU-A-0338221 ist bereits eine aus mindestens zwei Schichten bestehende Folienbahn bekannt, die aus mindestens einer ein- oder mehrschichtigen Kunststoffolienbahn oder Papierbahn besteht, mit einer Gesamtdicke von 50 bis 500 µm, vorzugsweise 100 bis 400 µm, sowie einer unmittelbar darauf oder über eine darauf befestigte Zwischenschicht oder Kunststoffzwischenschicht angeordneten 3 bis 30 µm dicken, vorzugsweise 5 bis 10 µm dicken, unter Mitverwendung mindestens eines monomeren, di- und/oder trimeren Acrylates hergestellten Acrylat-Lackschicht zusammengesetzt ist.

Gemäß EU-A-0338221 besteht die strahlenhärtbare Lackschicht aus einem Mattlack auf der Basis eines Acrylatgemisches, das durch Umsetzung mindestens eines prepolymeren Acrylates mittleren Molekulargewichtes von 500 bis 2000 mit dem monomeren, di- und/oder trimeren Acrylat hergestellt ist. Der Lack enthält je 100 Gew.-Teile des Acrylatlackes (berechnet ohne jedes Zusatzmittel, Verarbeitungshilfsmittel oder Treibmittel) 20 bis 60 Gew.-Teile eines Mattierungsmittelgemisches, 1 bis 10 Gew.-Teile eines UV-Initiators oder UV-Initiatorgemisches, 0 bis 10 Gew.-Teile eines Treibmittels oder Treibmittelgemisches, wobei in dem Mattlack keine zusätzlichen Verdünnungs- oder Lösungsmittel enthalten sind, das eingesetzte monomere, di- und/oder trimere Acrylat eine Viskosität von 7 bis 200 mPas und das verwendete prepolymere Acrylat eine Viskosität von 500 bis 15000 mPas aufweisen und das Mattierungsmittelgemisch aus mindestens zwei chemisch verschiedenen Mattierungsmitteln und/oder Mattierungsmitteln mit unterschiedlichen Teilchengrößen besteht. Diese Einstellung des Mattgrades mit Mattierungsmitteln hat jedoch den Nachteil, daß eine Reaktionsverzögerung eintritt und ein erhöhter Energieaufwand durch Erhitzung bzw. Erwärmung der Lackschicht bei der Aushärtung zusätzlich erforderlich wird und daß die Kratzfestigkeit durch die Mattierungsmittel (Schreibeffekt bei sehr matten Einstellungen) herabgesetzt wird.

Ziel und Aufgabe der vorliegenden Erfindung war es daher, den Zusatz von Mattierungsmitteln bei diesen Acrylatlacken zu vermindern oder ohne Mattierungsmittel auszukommen und ein verbessertes Verfahren zur Herstellung der UV-härtbaren Lackbeschichtung zu erreichen.

Erfindungsgemäß wurde festgestellt, daß diesen Zielen und Aufgaben ein mit UV-härtbarem Lack beschichtetes flächenförmiges Trägermaterial mit matter oder glänzender Oberfläche gerecht wird, wobei das Trägermaterial aus mindestens einer ein- oder mehrschichtigen Kunststoffolienbahn oder Papierbahn mit einer Gesamtdicke von 80 bis 500 µm besteht und unmittelbar darauf oder über eine darauf befestigte Zwischenschicht oder Kunststoffzwischenschicht eine strahlenhärtbare Lackschicht aufgebracht wird, die mindestens ein monomeres Acrylat (mono-, di-, tri- und/oder tetrameres Acrylat) sowie gegebenenfalls übliche Zusatzstoffe enthält, wobei die strahlenhärtbare Lackschicht aus einem Lack auf der Basis eines Acrylatgemisches besteht, das durch Umsetzung mindestens eines prepolymeren Acrylates mittleren Molekulargewichtes von 500 bis 2000 mit dem monomeren Acrylat (mono-, di-, tri- und/oder tetrameren Acrylat) hergestellt wird, und der Lack je 100 Gew.-Teile des Acrylatlackes (berechnet ohne jedes Zusatzmittel oder Verarbeitungshilfsmittel), 1 bis 10 Gew.-Teile eines UV-Initiators oder UV-Initiatorgemisches enthält oder aus diesem Bestandteil besteht und daß die aufgebrachte Lackschicht anschließend durch UV-Strahlen ausgehärtet wird. Gemäß der Erfindung wird der Mattgrad des UV-Lackes durch Zugabe von mono- und/oder difunktionellen Monomeren eingestellt und für die UV-Härtung werden mehrere Strahlenquellen mit definiertem Wellenlängenbereich verwendet.

Nach der vorliegenden Erfindung ist es besonders wichtig, daß durch mindestens eine UV-Strahlenquelle oder mindestens einen UV-Strahler, vorzugsweise die erste oder ersten UV-Strahlenquelle(n) einen Wellenlängenbereich von 100 bis 200 nm UV-Strahlen mit niedrigen Wellenlängen, vorzugsweise 130 bis 190 nm, umfaßt, die mit der strahlenhärtbaren Lackschicht beschichtete Bahn bestrahlt wird. Durch diese UV-Strahlenquelle mit diesem Wellenlängenbereich gelingt die Einstellung des Mattgrades mit verminderter Mattierungsmittelmenge oder ohne Mattierungsmittel.

In Vorbereitung der vorliegenden Erfindung wurden zahlreiche Versuche gemacht, die die Zusammensetzung der Lackschicht, die Wellenlänge und dergleichen und deren Variation betraf. Bei diesen Vorversuchen wurde zunächst die mit dem strahlenhärtbaren Lack beschichtete Kunststoffbahn einer oder mehrerer UV-Strahlenquelle(n) (Standard HG-Strahler) mit UV-Strahlen höherer Wellenlänge, u.a. mehr als 200 nm, vorzugsweise 200 bis 800 nm bestrahlt. Auch dabei konnte ein Mattierungseffekt erzielt werden, es traten jedoch die Nachteile auf daß hohe Anteile an Mattierungsmittel erforderlich werden, ein Schreibeffekt an der Oberfläche entsteht und die Viskosität des Lackes durch die hohen Mattierungsmittelanteile sehr negativ beeinflußt werden.

Erst durch die erfindungsgemäße Kombination der Zusammensetzung, der Verwendung von UV-Strahlen niedriger Wellenlänge und vorzugsweise auch einer nachfolgenden Behandlung der strahlenhärtbaren Lackschicht mit UV-Strahlen höherer Wellenlänge gelingt es eine verbesserte Kratzfestigkeit der Lackbeschichtung zu erzielen und die gewünschte Mattierung mit einem verminderten Mattierungsmittelgehalt zu erhalten oder sogar UV-Lacke ohne Mattierungsmittel (wie Si0₂, Talkum, Wachse) zu mattieren.

Nach der bevorzugten Ausführungsform der Erfindung wirkt daher durch die erste(n) UV-Strahlenquelle(n) ein monochromatisches Licht im Bereich zwischen 100 bis 200 nm (UV-Strahlen niedriger Wellenlänge), vorzugsweise UV-Licht mit einer Wellenlänge von 130 bis 190 nm, besonders bevorzugt mit einer Wellenlänge von 172 nm, auf die beschichtete Bahn ein und die mit der strahlenhärtbaren Lackschicht beschichtete Bahn wird nachfolgend einer UV-Bestrahlung mit Strahlen einer höheren Wellenlänge unterworfen.

Nach der bevorzugten Ausführungsform wird die mit der strahlenhärtbaren Lackschicht beschichtete Bahn zuerst zu der oder den UV-Strahlenquelle(n) oder UV-Strahler(n) mit Strahlen niedriger Wellenlänge geleitet und nachfolgend zu einem oder mehreren UV-Strahlern mit UV-Strahlen mit einer höheren Wellenlänge transportiert, dessen oder deren UV-Strahlen um mehr als 2 nm, vorzugsweise 10 nm oberhalb des Lichtspektrums bzw. der Wellenlänge der Strahlen des oder der ersten UV-Strahler(s) liegt und eine Wellenlänge von mindestens 200 nm aufweisen.

Nach einer weiteren bevorzugten Ausführungsform des erfindungsgemäßen Verfahrens wirkt bzw. wirken der oder die nachfolgende(n) UV-Strahler mit Strahlen höherer Wellenlänge auf die beschichtete Bahn ein, so daß die mit einer strahlenhärtbaren Lackschicht beschichtete Bahn mit Strahlen im Wellenlängenbereich zwischen 200 und 900 nm bestrahlt wird.

Nach einer anderen bevorzugten Ausführungsform weist oder weisen der oder die auf die UV-Strahler mit Strahlen niedriger Wellenlänge folgende(n) Strahler (Strahler mit höherer Wellenlänge) ein monochromatisches UV-Licht zwischen 220 bis 450 nm, vorzugsweise 308 nm, auf und die mit einer strahlenhärtbaren Lackschicht beschichtete Bahn wird während des Transportes mit Strahlen dieser Wellenlänge(n) bestrahlt.

Nach einer weiteren bevorzugten Ausführungsform wird die Strahlenhärtung unter ultraviolettem Licht bei Folientemperaturen unter 70 °C, vorzugsweise zwischen 7 und 60 °C, durchgeführt.

Nach einer bevorzugten Ausführungsform beträgt das Gewichtsverhältnis der verwendeten mono- und/oder difunktionellen monomeren Acrylate zu den prepolymeren sowie tri- und tetrafunktionellen monomeren Acrylaten 1: 0,001 bis 1 : 1,5, vorzugsweise 1 : 0,1 bis 1 : 1.

Bei dem Aufbringen der Lackschicht wird bevorzugt eine durchschnittliche Dicke von 2 bis 25 µm, vorzugsweise 3 bis 15 µm, eingehalten oder eingestellt.

Nach einer anderen bevorzugten Ausführungsform wird der Lack bei der Verwendung von prepolymeren Acrylaten mit einer höheren Viskosität auf Temperaturen bis zu 80 °C, vorzugsweise bis zu 70 °C, vor dem Auftragen bzw. Beschichten erwärmt.

Nach einer weiteren bevorzugten Ausführungsform werden dem UV-Lack weniger als 6 Gew.-% zusätzliches Verdünnungsmittel oder Lösungsmittel, vorzugsweise keine Verdünnungsmittel und/oder Lösungsmittel zugefügt.

Vorzugsweise werden dem Lack noch chemische Härtungsmittel oder Härtungsbeschleuniger zugesetzt. Nach einer bevorzugten Ausführungsform wird zu den monomeren und prepolymeren Acrylaten ein polyfunktionelles aliphatisches lösungsmittelfreies Polyisocyanat 100 % in Gewichtsmengen von 1 bis 15 Gew.-Teilen, vorzugsweise 2 bis 7,5 Gew.-Teilen, zugesetzt.

Nach einer vorzugsweisen Ausführungsform werden zu dem UV-Lack weniger als 25 Gew.-% (bezogen auf den Gesamtlack) Mattierungsmittel, vorzugsweise weniger als 10 Gew.-%, besonders bevorzugt keine Mattierungsmittel zugefügt.

Gemäß der Erfindung wird die Mattstufe des UV-Lackes durch Mischen von Lack ohne mono- und difunktionelle Monomeren mit Lack und hohem Anteil an mono- und difunktionellen Monomeren eingestellt.

Als Mattierungsmittel sind anorganisch-chemische und/oder organisch-chemische Mattierungsmittel in dem Acrylatlack innerhalb der angegebenen Gewichtsmengen enthalten, vorzugsweise ein unpolares, anorganisch-chemisches Mattierungsmittel oder unpolares, anorganisch-chemisches Mattierungsmittelgemisch. Bevorzugt werden jedoch sehr geringe Mattierungsmittelmengen, vorzugsweise weniger als 10 Gew.-%, besonders bevorzugt keine Mattierungsmittel, eingesetzt.

Die anorganisch-chemischen Mattierungsmittel weisen in der Lackschicht eine mittlere Korngröße von 0,5 bis 20 µm, vorzugsweise 2 bis 12 µm, auf, während die organischchemischen Mattierungsmittel eine mittlere Teilchengröße von 0,04 bis 10 µm, vorzugsweise 0,2 bis 5 µm besitzen.

Als prepolymere Acrylate werden Polyesteracrylate, Urethanacrylate, Acrylacrylate und/oder Methacrylacrylate, Oligoesteracrylate, Epoxiacrylate und/oder Mischungen von zwei oder mehreren dieser prepolymeren Acrylate bevorzugt eingesetzt, wobei das durchschnittliche Molekulargewicht der prepolymeren Acrylate zwischen 500 bis 2000 liegt.

Gemäß der Erfindung wird die Lackschicht durch Reaktion somit von mindestens einem monomeren Acrylat mit mindestens einem prepolymeren Acrylat gebildet, wobei diese Bestandteile bevorzugt vor dem Auftragen auf die Trägerbahn vermischt werden.

Als Trägerbahn werden als Kunststoffolienbahn an sich bekannte Kunststoffe, wie z. B. solche auf der Basis oder unter Mitverwendung von Polyolefinen, Polyvinylchlorid, Polyvinylidenchlorid, Ethylen-Propylen-Dien-Mischpolymerisaten, Polymerisate, die Styrol, Acrylnitril, Polycarbonate, Fluorpolymerisate und/oder Polyester enthalten oder daraus bestehen, eingesetzt.

Nach einer bevorzugten Ausführungsform wird als Trägerbahn eine Polyolefinfolie verwendet, die vorzugsweise mit durch Oberflächenaktivierung verbesserbare Haftungseigenschaften aufweist. Das Polyolefin ist dabei bevorzugt Polyethylen, Polypropylen, ein Mischpolymeres von Polyethylen oder Polypropylen, ein Copolymeres von Polyethylen oder Polypropylen mit einer Wärmebeständigkeit VSP/A übere 100 °C oder ein Gemisch davon. Nach einer Ausführungsform enthält der Kunststoff für die Trägerbahn zusätzlich 1 bis 50 Gew.-% feinteilige Cellulose, mit mittlerem Teilchendurchmesser von 1 bis 100 µm, wobei die größte mittlere Länge der Cellulose bis 300 µm beträgt und/oder der Kunststoff enthält zusätzlich
a) 1 bis 50 Gew.-% eines mineralischen Füllstoffs und/oder
b) 0,5 bis 20 Gew.-%, jeweils bezogen auf die Summe von Polyolefin und Cellulose, eines Modifizierungsmittels aus der Reihe der Polymeren auf der Basis von Styrol und Elastomeren und der Copolymerisate oder Pfropfcopolymerisat von Ethylen mit reaktiven Monomeren sowie
c) Pigmente und/oder Farbstoffe in üblicher Menge.

Nach einer bevorzugten Ausführungsform enthält diese Trägerfolie auf Polyolefinbasis 3 bis 30 Gew.-% Cellulose und/oder Glimmer, Talkum, Kieselsäure, Silikate und/oder Ti0₂ oder andere Füllstoffe.

Im Rahmen der Erfindung geeignet sind feinteilige reine oder weniger reine, native oder regenerierte Cellulose. Beispielsweise sind geeignet α-Cellulose, die bevorzugt wird, β-Cellulose, γ-Cellulose, Baumwolle mit einem Cellulosegehalt von mindestens 80 % oder regenerierte Cellulose.

Die Cellulosemenge liegt zwischen 1 und 50 Gew.-%, bezogen auf die Summe von Polyolefin und Cellulose. Wird ein Anteil von 50% überschritten, so verliert das Produkt zunehmend den Foliencharakter und ein papierähnlicher Charakter tritt in den Vordergrund.

Unter 1 % ist die erzielte Verbesserung der Oberflächenaktivierung nicht ausgeprägt genug. Bevorzugt enthält die Folie 3 bis 30 Gew.-% Cellulose. Der Feinheitsgrad der Cellulose in der erfindungsgemäßen Folie liegt zwischen 1 und 100 µm. Ein feinerer Verteilungsgrad ist aufwendig, bei größeren Partikeln treten Probleme bei der Herstellung glatter Folien auf. Bei faserförmiger Cellulose können jedoch auch Teilchen bis zu 5 detex einer größten mittleren Länge von 300 µm verwendet werden, ohne daß hinsichtlich der Folienbildung grundsätzlich Schwierigkeiten auftreten.

Als Polyolefin werden im Rahmen der Erfindung verwendet alle Arten von Polyethylen, einschließlich Polyethylen hoher Dichte (HDPE), Polyethylen niedriger Dichte (LDPE), Polypropylen, Mischpolymere und Ethylen und Propylen sowie die Copolymeren von Ethylen oder Propylen, welche eine Wärmebeständigkeit VSP A über 100 °C aufweisen sowie Gemische dieser Stoffe und/oder Elastomere. Bei den Copolymeren kommen vor allem solche mit Vinylverbindungen in Betracht.

Die erfindungsgemäße Trägerfolie kann zusätzlich auch mineralische Füllstoffe enthalten. Derartige Füllstoffe für Polymerfolien sind bekannt. Besonders geeignet und daher bevorzugt werden Glimmer, Talkum, Silikate und Kieselsäure in ihren verschiedenen Formen. Beispiele für andere brauchbare mineralische Füllstoffe sind Carbonate, insbesondere Calciumcarbonate, wie Kalkstein und Kreide sowie Magnesiumcarbonate und dergleichen.

Durch den Zusatz geeigneter mineralischer Füllstoffe lassen sich Eigenschaften wie Vicatpunkt, Shorehärte und Zugfestigkeit beeinflussen. Falls derartige mineralische Füllstoffe vorhanden sind, enthält die Folie 1 bis 50 Gew.-%, vorzugsweise 2 bis 30 Gew.-%, bezogen auf die Summe von Polyolefin und Cellulose.

Außerdem kann die Folie noch ein oder mehrere organische Modifizierungsmittel enthalten. Diese dienen zur Regelung von Zähigkeit, Kalandrierbarkeit, Extrudierbarkeit und ähnliche Eigenschaften. Eine bevorzugte Gruppe hierfür sind Blockpolymer von Styrol mit Butadien oder Isobutylen oder Isopren.

Andere geeignete Modifizierungsmittel sind Polymerisate auf Basis Styrol-Butadien, Methacrylat-Butadien-Styrol-Polyolefine, die funktionelle Gruppen enthalten, eignen sich besonders zur Beeinflussung des Verhältnisses von physikalischen Eigenschaften zur Verklebbarkeit. Derartige Modifizierungszusätze sind in einer Menge von 0,5 bis 20 Gew.-%, vorzugsweise von 2 bis 10 Gew.-%, bezogen jeweils auf die Summe von Polyolefin und Cellulose, vorhanden.

Wie bereits erwähnt, kommen die Vorteile der erfindungsgemäßen Folie dann zur Geltung, wenn sie einer die Oberflächenadhäsion verbessernden Behandlung unterworfen wird. Bevorzugt besteht diese Oberflächenbehandlung in einer Aktivierung durch Bestrahlung. Unter den Bestrahlungsmethoden wird wiederum eine Coronabehandlung bevorzugt.

Nach einer bevorzugten und vorteilhaften Ausführungsform enthält die Trägerfolie oder mindestens eine Oberfläche oder Oberflächenschicht der Trägerfolie reaktive Gruppen. Die reaktiven Gruppen der Trägerfolie bestehen nach der bevorzugten Ausführungsform aus Hydroxy- und/oder Methoxy-Gruppen und/oder N-Methylol-Gruppen ((=N-CH₂-OH)-Gruppen) und/oder ((=N-CH₂-OR)-Gruppen). Die auf mindestens einer Oberfläche oder Oberflächenschicht Hydroxy-Gruppen enthaltenden Kunststoffolien bestehen nach einer bevorzugten Ausführungsform aus Cellulose, Cellulosederivaten und/oder Stärke sowie einem Olefinhomo- und/oder -copolymerisat oder Vinylchloridhomo-, -co- oder - pfropfpolymerisat.

Als Stärke sind die an sich bekannten Stärkesorten, z.B. Kartoffelstärke und dergleichen geeignet sowie chemisch und/oder physikalisch modifizierte, feinteilige bzw. feinkörnige Stärkearten und Stärkeabbauprodukte. Bevorzugt werden jedoch feinstteilige bzw. feinkörnige Knollenstärken (insbesondere Kartoffelstärke), Getreidestärken (insbesondere Maisstärke) und/oder Wurzelstärken eingesetzt. Die Stärke kann nach einer Ausführungsform teilweise durch Lignin ersetzt werden. Nach einer bevorzugten Ausführungsform gelangen feinstteilige bzw. feinkörnige Gemische von Cellulose und Stärke zur Anwendung. Hydroxy-Gruppen anderer Verbindungen, z. B. von Vinylalkoholen, ergeben nicht so haftfeste Verbindungen innerhalb der erfindungsgemäßen Kombination.

Die auf mindestens einer Oberfläche oder Oberflächenschicht enthaltenden reaktiven Gruppen der Trägerfolie bestehen nach einer anderen Ausführungsform aus Hydroxygruppen, Carboxylgruppen, Methoxy- und/oder N-Methylolgruppen ((=N-CH₂-OH)-Gruppen) und/oder ((=N-CH₂-OR)-Gruppen), die vorzugsweise unter Mitverwendung von Hexamethoxy-Methylmelamin und/oder nichtplastifiziertem Melaminharz oder von Umsetzungsprodukten dieser Verbindungen, vor, bei der, nach der Herstellung der Kunststoffe oder Kunststoffolien auf der Basis von Olefinhomo- und/oder -copolymerisat oder Vinylchloridhomo-, -co- oder -pfropfpolymerisat zugefügt werden.

Nach einer bevorzugten Ausführungsform besteht die Trägerbahn aus einer reaktive Verbindungen oder reaktive Gruppen enthaltenden Polyolefinfolie oder Polyolefinfolienbahn, wobei in der Polyolefinfolie oder Polyolefinfolienbahn ein feinteiliger Füllstoff oder ein Füllstoffgemisch sowie gegebenenfalls Verarbeitungshilfsmittel, Farbpigmente und/oder Modifizierungsmittel enthalten sind. Gemäß der Erfindung besteht die mindestens zwei-, vorzugsweise mehrschichtige Folie oder Folienbahn aus einer Polyolefinfolie oder Polyolefinfolienbahn, enthaltend ein Gemisch oder eine Legierung aus 70 bis 97,5 Gew.-Teilen, vorzugsweise 75 bis 85 Gew.-Teilen, eines Propylenhomo- oder -copolymerisates, und 30 bis 2,5 Gew.-Teilen, vorzugsweise 25 bis 15 Gew.-Teilen, Niederdruckpolyethylen, Polyvinylalkohol, Ethylen-Vinylalkohol-Copolymerisat, Ethylen-Carbonsäure-Copolymerisat, Ethylen-Acrylsäureester-Acrylsäure-Copolymerisat (EAA) oder -terpolymerisat oder Polycaprolacton oder Mischungen bzw. Legierungen von Niederdruckpolyethylen mit ein oder mehreren der vorgenannten Polymerisate, Co- oder Terpolymerisate und bezogen auf 100 Gew.-Teile der Kunststoffmischung bzw. -legierung 50 bis 150 Gew.-Teile, vorzugsweise 70 bis 120 Gew.-Teile, eines feinteiligen mineralischen Füllstoffes oder mineralischen Füllstoffgemisches, vorzugsweise Calciumcarbonat, Mikrotalkum, Kaolin und/oder Kieselsäuregel, von dem mehr als 60 Gew.-%, vorzugsweise mehr als 75 Gew.-%, (bezogen auf 100 Gew.-% eingesetzte bzw. enthaltende Füllstoffe) mindestens eine reaktive Substanz besitzen, wobei als reaktive Substanz mindestens eine polare und unpolare Gruppen aufweisende Substanz in Gewichtsmengen von 0,5 bis 10 Gew.-%, vorzugsweise 1 bis 5 Gew.-%, (bezogen auf 100 Gew.-Teile Füllstoff und Füllstoffgemisch) enthalten ist, die vorzugsweise auf der Oberfläche oder auf einem Teil der Oberfläche des Füllstoffes oder Füllstoffgemisches angeordnet ist und eine Oberflächenschicht oder Oberflächenfolie mit einer mittleren Dicke von 3 bis 30 µm, vorzugsweise 5 bis 20 µm, die auf bzw. über der Polyolefinfolie oder Polyolefinfolienbahn angeordnet ist. Die Polyolefinfolie oder Polyolefinfolienbahn weist eine Dicke von 30 bis 700 µm, vorzugsweise 70 bis 500 µm, auf.

Unter Verwendung der Erfindung lassen sich Folien mit einer hohen Oberflächenspannung und/oder einer guten Haftfestigkeit zu der darüber befindlichen Schicht und einer guten Bedruckbarkeit erzielen sowie einer ausreichenden Lagerzeit, innerhalb der die Oberflächenspannung nicht oder nur unwesentlich abnimmt.

Die Folie oder Folienbahn gemäß der Erfindung weist auf ihrer Oberfläche eine Oberflächenspannung von mehr als 68 dyn/cm, vorzugsweise von mehr als 71 dyn/cm, auf und/oder ist coronabehandelt oder durch ein Plasmaverfahren vorbehandelt, wobei die vorgenannten Werte der Oberflächenspannung eingestellt werden.

Gemäß einer bevorzugten Ausführungsform sind 0,1 bis 20 Gew.-Teile, vorzugsweise 1 bis 10 Gew.-Teile, des mit einer polaren Substanz versehenen Füllstoffes oder Füllstoffgemisches durch die gleiche Gewichtsmenge eines polaren Füllstoffes oder polaren Füllstoffgemisches, vorzugsweise ungecoatetes Kaolin und/oder Kieselsäuregel, ersetzt. Dadurch können je nach eingesetztem polaren Füllstoff höhere Oberflächenspannungen erzielt werden.

Nach einer vorzugsweisen Ausführungsform ist zwischen der Oberflächenschicht oder Oberflächenfolie und der Polyolefinfolie oder Polyolefinfolienbahn (als Unterfolie) eine Druckschicht angeordnet.

Als polare Substanz bzw. Substanzen ist bzw. sind mindestens eine organisch-chemische Verbindung mit mindestens einer OH-Gruppe und/oder COOH-Gruppe und/oder SiOR oder SiOR-Gruppe oder ähnliche Polysiloxangruppen und mindestens einer oder mehreren unpolaren Gruppen an der Oberfläche des mineralischen Füllstoffes enthalten und/oder dieser ist damit in Form einer dünnen Schicht ganz oder teilweise überzogen, wobei vorzugsweise die polaren Gruppen zum Füllstoff orientiert sind.

Bevorzugt werden als polare und unpolare Gruppen aufweisende Verbindungen gesättigte und ungesättigte Carbonsäuren mit einer C-Zahl über 3, vorzugsweise über 10, Hydroxycarbonsäure, Polyoxycarbonsäure mit einer C-Zahl über C₆, vorzugsweise über C₁₀, insbesondere Stearinsäure, Hydroxystearinsäure oder deren Ester und/oder Polysiloxane (z.B. Polydimethylsiloxan) oder Hydroxy- oder Amin-Gruppen aufweisende Polysiloxane oder eine oder mehrere Hydroxygruppen enthaltende Polysiloxane eingesetzt.

Nach einer bevorzugten Ausführungsform wird die erste Strahlenquelle unter Inertgas, vorzugsweise Stickstoff, betrieben.

Nach einer anderen bevorzugten Ausführungsform enthält das eingesetzte Prepolymer kein Verdünnungsmittel (auch keine monomeren Acrylate, d.h. 100 % Prepolymeres) und das Prepolymere wird mit einer Viskosität von 0,1 bis 200 Pas bei 23 °C, vorzugsweise 0,15 bis 50 Pas, eingesetzt.

Nach der bevorzugten Ausführungsform weist somit der zweite und/oder nachfolgende UV-Strahler eine höhere Leistung und/oder Strahlenintensität auf als der erste Strahler. Die mit der strahlenhärtbaren Lackschicht beschichtete Kunststoffbahn wird somit zuerst zu mindestens einem UV-Strahler mit UV-Strahlen niedriger Wellenlängen (100 bis 200 nm, vorzugsweise 130 bis 190 nm) transportiert und nachfolgend zu mindestens einer weiteren Strahlenquelle mit UV-Strahlen höherer Wellenlängen (200 bis 900 nm, jedoch mehr als 2 nm, vorzugsweise mehr als 10 nm oberhalb der Wellenlänge des oder der ersten UV-Strahler).

Nach einer bevorzugten Ausführungsform wird das Verfahren kontiniuerlich durchgeführt, indem die aus der Breitschlitzextruderdüse austretende Folienbahn oder Verbundfolienbahn nach an sich bekannten Verfahren, neben Chillroll- und/oder Walzen- oder Kalandrierverfahren, mit dem erfindungsgemäßen strahlenhärtbaren Lack beschichtet und nachfolgend weiter transportiert und nach dem erfindungsgemäßen Verfahren bestrahlt wird.

Nach einer weiteren Ausführungsform des erfindungsgemäßen Verfahrens ist nach dem bzw. den Strahler(n) mit dem UV-Spektrum von 100-200 nm, vorzugsweise ein monochromatischer Strahler mit 172 nm, ein Elektronenstrahler angeordnet und/oder der oder die UV-Strahler mit einer höheren Wellenlänge (über 200 nm) und höheren Leistung und/oder Strahlungsintensität ist bzw. sind ganz oder teilweise durch mindestens einen Elektronenstrahler ersetzt.

| **Beispiele** | | |
|---|---|---|
| Beispiel 1 | 52 Teile | 1,6 Hexandioldiacrylat (HDDA) |
| | 12 Teile | Trimethylolpropan-Triacrylat (TMPTA) |
| | 36 Teile | Polyesteracrylat |
| | 5 Teile | 1-Hydroxycyclohexylacetophenon |
| | 0,05 Teile | Slipmittel |
| | | Glanzgrad nach Gardener 60°: 2,5-5 |
| Beispiel 2 | 20 Teile | Tripropylenglycol-Diacrylat (TPGDA) |
| | 24 Teile | Trimethylolpropanethoxy-Triacrylate (TMPEOTA) |
| | 16 Teile | Polyetheracrylat |
| | 40 Teile | Aliphatisches Urethanacrylat |
| | 2 Teile | 1-Hydroxycyclohexylacetophenon |
| | 2 Teile | Benzophenon |
| | 5 Teile | Aliphatisches Polyisocyanat 100 % |
| | | Glanzgrad nach Gardener 60°: 10-15 |
| Beispiel 3 | 10 Teile | Tripropylenglycol-Diacrylat (TPGDA |
| | 25 Teile | Oligotriacrylate (OTA) |
| | 20 Teile | Polyetheracrylat |
| | 45 Teile | Polyesteracrylat |
| | 2 Teile | 1-Hydroxycyclohexylacetophenon |
| | 7,5 Teile | Aliphatisches Polyisocyanat 100 % |
| | 0,02 Teile | Slipmittel |
| | | Glanzgrad nach Gardener 60°: 20-30 |

## Patentansprüche

1. Verfahren zur Herstellung eines mit UV-härtbarem Lack beschichteten flächenförmigen Trägermaterials mit matter oder glänzender Oberfläche, wobei das Trägermaterial aus mindestens einer ein- oder mehrschichtigen Kunststoffolienbahn oder Papierbahn mit einer Gesamtdicke von
80 bis 500 µm
besteht und unmittelbar darauf oder über eine darauf befestigte Zwischenschicht oder Kunststoffzwischenschicht eine strahlenhärtbare Lackschicht aufgebracht wird, die mindestens ein monomeres Acrylat (mono-, di-, tri- und/oder tetrameres Acrylat) sowie gegebenenfalls übliche Zusatzstoffe enthält, wobei die strahlenhärtbare Lackschicht aus einem Lack auf der Basis eines Acrylatgemisches besteht, das durch Umsetzung mindestens eines prepolymeren Acrylates mittleren Molekulargewichtes von 500 bis 2000 mit dem monomeren Acrylat (mono-, di-, tri- und/oder tetrameren Acrylat) hergestellt wird, und der Lack je 100 Gew.-Teile des Acrylatlackes (berechnet ohne jedes Zusatzmittel oder Verarbeitungshilfsmittel), 1 bis 10 Gew.-Teile eines UV-Initiators oder UV-Initiatorgemisches enthält und daß die aufgebrachte Lackschicht anschließend durch UV-Strahlen ausgehärtet wird, dadurch gekennzeichnet, daß der Mattgrad des UV-Lackes durch Zugabe von mono- und/oder difunktionellen Monomeren eingestellt wird, und daß für die UV-Härtung mehrere Strahlenquellen mit definiertem Wellenlängenbereich verwendet werden, wobei durch mindestens eine UV-Strahlenquelle oder mindestens einen UV-Strahler die mit der strahlenhärtbaren Lackschicht beschichtete Bahn zuerst mittels UV-Strahlen mit einem Wellenlängenbereich von
100 bis 200 nm (UV-Strahlen mit niedrigen Wellenlängen)
bestrahlt wird.

2. Verfahren nach Anspruch 1, dadurch gekennzeichnet, daß durch mindestens eine UV-Strahlenquelle oder mindestens einen UV-Strahler die mit der strahlenhärtbaren Lackschicht beschichtete Bahn zuerst mittels UV-Strahlen mit einem Wellenlängenbereich von
130 bis 190 nm
bestrahlt wird.

3. Verfahren nach einem oder mehreren der Ansprüche 1 bis 2, dadurch gekennzeichnet, daß durch die erste(n) UV-Strahlenquelle(n) ein monochromatisches Licht im Bereich zwischen 100 bis 200 nm (UV-Strahlen niedriger Wellenlänge), vorzugsweise UV-Licht mit einer Wellenlänge von 172 nm, auf die beschichtete Bahn einwirkt und die mit der strahlenhärtbaren Lackschicht beschichtete Bahn nachfolgend einer UV-Bestrahlung mit Strahlen einer höheren Wellenlänge unterworfen wird.

4. Verfahren nach einem oder mehreren der Ansprüche 1 bis 3, dadurch gekennzeichnet, daß die mit der strahlenhärtbaren Lackschicht beschichtete Bahn zuerst zu der oder den UV-Strahlenquelle(n) oder UV-Strahler(n) mit Strahlen niedriger Wellenlänge geleitet und nachfolgend zu einem oder mehreren UV-Strahlern mit UV-Strahlen mit einer höheren Wellenlänge transportiert wird, dessen oder deren UV-Strahlen um mehr als 2 nm, vorzugsweise 10 nm oberhalb des Lichtspektrums bzw. der Wellenlänge der Strahlen des oder der ersten UV-Strahler(s) liegt und eine Wellenlänge von mindestens 200 nm aufweisen.

5. Verfahren nach einem oder mehreren der Ansprüche 1 bis 4, dadurch gekennzeichnet, daß der oder die nachfolgende(n) UV-Strahler mit Strahlen höherer Wellenlänge auf die beschichtete Bahn einwirken, so daß die mit einer strahlenhärtbaren Lackschicht beschichtete Bahn mit Strahlen im Wellenlängenbereich zwischen
200 und 900 nm
bestrahlt wird.

6. Verfahren nach einem oder mehreren der Ansprüche 1 bis 5, dadurch gekennzeichnet, daß der oder die auf die UV-Strahler mit Strahlen niedriger Wellenlänge folgende(n) Strahler ein monochromatisches UV-Licht zwischen
220 bis 450 nm, vorzugsweise
308 nm,
aufweist bzw. aufweisen und die mit einer strahlenhärtbaren Lackschicht beschichtete Bahn während des Transportes mit Strahlen dieser Wellenlänge(n) bestrahlt wird.

7. Verfahren nach einem oder mehreren der Ansprüche 1 bis 6, dadurch gekennzeichnet, daß die Strahlenhärtung unter ultraviolettem Licht bei Folientemperaturen unter 70 °C, vorzugsweise zwischen 7 und 60 °C, durchgeführt wird.

8. Verfahren nach einem oder mehreren der Ansprüche 1 bis 7, dadurch gekennzeichnet, daß das Gewichtsverhältnis der verwendeten mono- und/oder difunktionellen monomeren Acrylate zu den prepolymeren sowie tri- und tetrafunktionellen monomeren
1 : 0,001 bis 1 : 1,5, vorzugsweise
1 : 0,1 bis 1 : 1,1
beträgt.

9. Verfahren nach einem oder mehreren der Ansprüche 1 bis 8, dadurch gekennzeichnet, daß bei dem Aufbringen der Lackschicht eine durchschnittliche Dicke von
2 bis 25 µm, vorzugsweise
3 bis 15 µm,
eingehalten oder eingestellt wird.

10. Verfahren nach einem oder mehreren der Ansprüche 1 bis 9, dadurch gekennzeichnet, daß der Lack bei der Verwendung von prepolymeren Acrylaten mit einer höheren Viskosität auf Temperaturen
bis zu 80 °C, vorzugsweise
bis zu 70 °C,
vor dem Auftragen bzw. Beschichten erwärmt wird.

11. Verfahren nach einem oder mehreren der Ansprüche 1 bis 10, dadurch gekennzeichnet, daß zu den monomeren und prepolymeren Acrylaten ein polyfunktionelles aliphatisches lösungsmittelfreies Polyisocyanat 100 % in Gewichtsmengen von
1 bis 15 Gew.-Teile, vorzugsweise
2 bis 7,5 Gew.-Teile,
verwendet wird.

12. Verfahren nach einem oder mehreren der Ansprüche 1 bis 11, dadurch gekennzeichnet, daß zu dem UV-Lack weniger als 25 Gew.-% (bezogen auf den Gesamtlack) Mattierungsmittel, vorzugsweise keine Mattierungsmittel, zugefügt werden.

13. Verfahren nach einem oder mehreren der Ansprüche 1 bis 12, dadurch gekennzeichnet, daß die Mattstufe des UV-Lackes durch Mischen von Lack ohne mono- und difunktionelle Monomeren mit Lack mit hohem Anteil an mono- und difunktionellen Monomeren eingestellt wird.

14. Verfahren nach einem oder mehreren der Ansprüche 1 bis 13, dadurch gekennzeichnet, daß zu dem UV-Lack weniger als 6 Gew.-% zusätzliches Verdünnungsmittel oder Lösungsmittel, vorzugsweise kein Verdünnungs- und/oder Lösungsmittel, zugefügt werden.

15. Verfahren nach einem oder mehreren der Ansprüche 1 bis 14, dadurch gekennzeichnet, daß die erste(n) Strahlenquelle(n) unter Inertgas betrieben wird (werden), vorzugsweise Stickstoff

16. Verfahren nach einem oder mehreren der Ansprüche 1 bis 15, dadurch gekennzeichnet, daß Prepolymere verwendet werden, die kein Verdünnungsmittel (auch keine monomeren Acrylate, d.h. 100 % Prepolymere) enthalten, und Prepolymere mit einer Viskosität von 0,1 bis 200 Pas bei 23 °C, vorzugsweise 0,15 bis 50 Pas, eingesetzt werden.

17. Verfahren nach einem oder mehreren der Ansprüche 1 bis 16, dadurch gekennzeichnet, daß die nachfolgende(n) UV-Strahler (mit 202-900 nm) eine höhere Leistung und/oder Strahlungsintensität aufweist bzw. aufweisen als der bzw. die erste(n) UV-Strahler.

18. Verfahren nach einem oder mehreren der Ansprüche 1 bis 17, dadurch gekennzeichnet, daß nach dem ersten bzw. den ersten Strahler(n) mit dem UV-Spektrum von 100-200 nm, vorzugsweise ein monochromatischer Strahler mit 172 nm, ein Elektronenstrahler angeordnet ist, der den bzw. die UV-Strahler mit dem Wellenlängenbreich über 200 nm ersetzt.

## Claims

1. A method for the production of a planar support material having a matte or glossy surface, coated with UV-hardenable paint, wherein the support material consists of at least one single- or multi-layered plastics film web or paper web having a total thickness of
80 to 500 µm
and a radiation-hardenable paint layer is applied immediately thereon or over an intermediate layer or intermediate plastics layer fastened thereto, which paint layer contains at least one monomeric acrylate (monomeric, dimeric, trimeric and/or tetrameric acrylate) and also optionally conventional additives, the radiation-hardenable paint layer consisting of a paint on the basis of an acrylate mixture which is produced by reacting at least one prepolymeric acrylate of an average molecular weight of 500 to 2000 with the monomeric acrylate (monomeric, dimeric, trimeric and/or tetrameric acrylate), and the paint containing per 100 parts by weight of the acrylate paint (calculated without any additive or processing auxiliary) 1 to 10 parts by weight of a UV-initiator or UV-initiator mixture, and the applied paint layer is then hardened by UV radiation, characterised in that the degree of matte of the UV paint is adjusted by the addition of monofunctional and/or difunctional monomers, and that a plurality of radiation sources with defined wavelength ranges are used for the UV hardening, with the web coated with the radiation-hardenable paint layer first being exposed to UV rays having a wavelength range of
100 to 200 nm (UV rays with low wavelengths)
by at least one UV radiation source or at least one UV-emitter.

2. A method according to Claim 1, characterised in that the web coated with the radiation-hardenable paint layer is first exposed to UV rays having a wavelength range of
130 to 190 nm
by at least one UV radiation source or at least one UV-emitter.

3. A method according to one or more of Claims 1 or 2, characterised in that the first UV radiation source(s) cause(s) a monochromatic light in the range between 100 and 200 nm (UV rays of low wavelength), preferably UV light with a wavelength of 172 nm, to act on the coated web and the web coated with the radiation-hardenable paint layer is subsequently exposed to UV light with rays of a higher wavelength.

4. A method according to one or more of Claims 1 to 3, characterised in that the web coated with the radiation-hardenable paint layer is first passed to the UV radiation source(s) or UV-emitter(s) with rays of low wavelength and subsequently is transported to one or more UV-emitters with UV rays having a higher wavelength, the UV rays of which are more than 2 nm, preferably 10 nm, above the light spectrum or the wavelength of the rays of the first UV-emitter(s) and have a wavelength of at least 200 nm.

5. A method according to one or more of Claims 1 to 4, characterised in that the subsequent UV-emitter(s) act on the coated web with rays of a higher wavelength, so that the web coated with a radiation-hardenable paint layer is exposed to rays in the wavelength range of
200 to 900 nm.

6. A method according to one or more of Claims 1 to 5, characterised in that the emitter(s) following the UV-emitters with rays of low wavelength has/have a monochromatic UV light between
220 and 450 nm, preferably
308 nm,
and the web coated with a radiation-hardenable paint layer is exposed to rays of this/these wavelength(s) during transport.

7. A method according to one or more of Claims 1 to 6, characterised in that the radiation hardening is performed under ultraviolet light at film temperatures of less than 70°C, preferably between 7 and 60°C.

8. A method according to one or more of Claims 1 to 7, characterised in that the weight ratio of the monofunctional and/or difunctional monomeric acrylates used to the prepolymeric and trifunctional and tetrafunctional monomeric acrylates is
1 : 0.001 to 1 : 1.5, preferably
1 : 0.1 to 1 : 1.1.

9. A method according to one or more of Claims 1 to 8, characterised in that upon application of the paint layer an average thickness of
2 to 25 µm, preferably
3 to 15 µm,
is maintained or set.

10. A method according to one or more of Claims 1 to 9, characterised in that the paint when using prepolymeric acrylates having a relatively high viscosity is heated to temperatures of
up to 80°C, preferably
up to 70°C,
before application or coating.

11. A method according to one or more of Claims 1 to 10, characterised in that a polyfunctional aliphatic solvent-free polyisocyanate 100% is added to the monomeric and prepolymeric acrylates in quantities by weight of
1 to 15 parts by weight, preferably
2 to 7.5 parts by weight.

12. A method according to one or more of Claims 1 to 11, characterised in that less than 25% by weight (relative to the total paint) matting agent, preferably no matting agent, is added to the UV paint.

13. A method according to one or more of Claims 1 to 12, characterised in that the matte stage of the UV paint is adjusted by mixing paint without monofunctional and difunctional monomers with paint having a high content of monofunctional and difunctional monomers.

14. A method according to one or more of Claims 1 to 13, characterised in that less than 6% by weight additional diluent or solvent, preferably no diluent and/or solvent, is added to the UV paint.

15. A method according to one or more of Claims 1 to 14, characterised in that the first radiation source(s) is/are operated under inert gas, preferably nitrogen.

16. A method according to one or more of Claims 1 to 15, characterised in that prepolymers are used which contain no diluent (and no monomeric acrylates, i.e. 100% prepolymer), and prepolymers having a viscosity of 0.1 to 200 Pas at 23°C, preferably 0.15 to 50 Pas, are used.

17. A method according to one or more of Claims 1 to 16, characterised in that the subsequent UV-emitter(s) (at 202-900 nm) has/have a higher output and/or intensity of radiation than the first UV-emitter(s).

18. A method according to one or more of Claims 1 to 17, characterised in that an electron emitter which replaces the UV-emitter(s) with the wavelength range of above 200 nm is arranged after the first emitter(s) with the UV spectrum of 100-200 nm, preferably a monochromatic emitter at 172 nm.

## Revendications

1. Procédé pour la fabrication d'une matière de support de forme plate enduite d'une laque durcissable aux rayons ultraviolets, possédant une surface mate ou brillante, dans lequel la matière de support est constituée par au moins une bande de feuille en matière synthétique ou une bande de papier monocouche ou multicouche possédant une épaisseur totale de
80 à 500 µm
et on applique directement sur cette dernière ou par-dessus une couche intermédiaire ou une couche intermédiaire en matière synthétique fixée sur cette dernière, une couche de laque durcissable par exposition à un rayonnement qui contient au moins un acrylate monomère (un acrylate monomère, dimère, trimère et/ou tétramère), ainsi que le cas échéant des additifs habituels, la couche de laque durcissable par exposition à un rayonnement étant constituée par une laque à base d'un mélange d'acrylates que l'on prépare par mise en réaction d'au moins un acrylate prépolymère possédant un poids moléculaire moyen de 500 à 2000 avec l'acrylate monomère (acrylate monomère, dimère, trimère et/ou tétramère), la laque contenant, par 100 parties en poids de la laque d'acrylate (calculées en l'absence d'additif ou d'adjuvant de traitement respectif), de 1 à 10 parties en poids d'un initiateur du durcissement aux rayons ultraviolets ou d'un mélange d'initiateurs du durcissement aux rayons ultraviolets, et on durcit ultérieurement par exposition à un rayonnement ultraviolet la couche de laque appliquée, caractérisé en ce qu'on règle le degré de matage de la laque durcissable aux rayons ultraviolets par addition de monomères mono- et/ou difonctionnels et en ce qu'on utilise, pour le durcissement aux rayons ultraviolets, plusieurs sources de rayonnements possédant une gamme définie de longueurs d'ondes, dans lequel on expose à un rayonnement, va au moins une source de rayonnement ultraviolet ou via au moins un émetteur de rayonnement ultraviolet, la bande enduite avec la couche de laque durcissable par exposition à un rayonnement, d'abord à l'aide de rayons ultraviolets possédant une gamme de longueurs d'ondes de
100 à 200 nm (rayons ultraviolets de faibles longueurs d'ondes).

2. Procédé selon la revendication 1, caractérisé en ce qu'on expose à un rayonnement, va au moins une source de rayonnement ultraviolet ou via au moins un émetteur de rayonnement ultraviolet, la bande enduite avec la couche de laque durcissable par exposition à un rayonnement, d'abord à l'aide de rayons ultraviolets possédant une gamme de longueurs d'ondes de
130 à 190 nm.

3. Procédé selon une ou plusieurs des revendications 1 à 2, caractérisé en ce qu'on laisse agir, via la ou les premières sources de rayonnement ultraviolet, une lumière monochromatique dans la gamme entre 100 et 200 nm (rayons ultraviolets de faibles longueurs d'ondes), de préférence une lumière ultraviolette possédant une longueur d'onde de 172 nm, sur la bande enduite et on soumet ultérieurement la bande enduite avec la couche de laque durcissable par exposition à un rayonnement, à une exposition à un rayonnement ultraviolet avec des rayons possédant une longueur d'onde supérieure.

4. Procédé selon une ou plusieurs des revendications 1 à 3, caractérisé en ce qu'on guide la bande enduite avec la couche de laque durcissable par exposition à un rayonnement d'abord en direction de la ou des sources de rayonnement ultraviolet ou en direction du ou des émetteurs de rayonnement ultraviolet possédant des rayons de faible longueur d'onde, et on la transporte ultérieurement en direction d'un ou de plusieurs émetteurs de rayonnement ultraviolet dont les rayons ultraviolets possèdent une longueur d'onde supérieure, le ou les rayons ultraviolets se trouvant à plus de 2 nm, de préférence à 10 nm au-dessus du spectre de lumière, respectivement de la longueur d'onde des rayons du ou des premiers émetteurs de rayonnement ultraviolet, et présentant une longueur d'onde d'au moins 200 nm.

5. Procédé selon une ou plusieurs des revendications 1 à 4, caractérisé en ce que le ou les émetteurs de rayonnement ultraviolet ultérieurs agissent sur la bande enduite avec des rayons possédant une longueur d'onde supérieure, si bien que l'on expose à un rayonnement la bande enduite avec une couche de laque durcissable par exposition à un rayonnement avec des rayons dont la gamme de longueurs d'ondes se situe entre
200 et 900 nm.

6. Procédé selon une ou plusieurs des revendications 1 à 5, caractérisé en ce que le ou les émetteurs de rayonnement ultraviolet faisant suite au ou aux émetteurs de rayonnement ultraviolet possédant des rayons de longueur d'onde inférieure présente, respectivement présentent une lumière ultraviolette monochromatique entre
220 et 450 nm, de préférence
de 308 nm,
et on expose à un rayonnement la bande enduite avec une couche de laque durcissable par exposition à un rayonnement, au cours du transport, avec des rayons de cette ou de ces longueurs d'ondes.

7. Procédé selon une ou plusieurs des revendications 1 à 6, caractérisé en ce que le durcissement par exposition à un rayonnement est réalisé sous une lumière ultraviolette à des températures de la feuille inférieures à 70°C, de préférence entre 7 et 60°C.

8. Procédé selon une ou plusieurs des revendications 1 à 7, caractérisé en ce que le rapport pondéral des acrylates monomères mono- et/ou difonctionnels utilisés aux acrylates prépolymères et aux acrylates monomères tri- et tétrafonctionnels s'élève
de 1:0,001 à 1:1,5, de préférence
de 1:0,1 à 1:1,1.

9. Procédé selon une ou plusieurs des revendications 1 à 8, caractérisé en ce qu'au cours de l'application de la couche de laque, on maintient ou on règle une épaisseur moyenne de
2 à 25 µm, de préférence
de 3 à 15 µm.

10. Procédé selon une ou plusieurs des revendications 1 à 9, caractérisé en ce qu'on chauffe la laque avant l'application, respectivement avant l'enduction, lorsqu'on utilise des acrylates prépolymères possédant une viscosité supérieure, à des températures
allant jusqu'à 80°C, de préférence
allant jusqu'à 70°C.

11. Procédé selon une ou plusieurs des revendications 1 à 10, caractérisé en ce qu'on utilise, pour obtenir les acrylates monomères et prépolymères, un polyisocyanate polyfonctionnel aliphatique à 100%, exempt de solvant, dans des quantités pondérales de
1 à 15 parties en poids, de préférence
de 2 à 7,5 parties en poids.

12. Procédé selon une ou plusieurs des revendications 1 à 11, caractérisé en ce qu'on ajoute à la laque durcissable par exposition à un rayonnement ultraviolet, des agents de matage à concurrence de moins de 25% en poids (rapportés à la laque totale); de préférence on n'ajoute aucun agent de matage.

13. Procédé selon une ou plusieurs des revendications 1 à 12, caractérisé en ce qu'on règle le degré de matage de la laque durcissable par exposition à un rayonnement ultraviolet en mélangeant une laque exempte de monomères mono- et difonctionnels avec une laque possédant une fraction élevée de monomères mono- et difonctionnels.

14. Procédé selon une ou plusieurs des revendications 1 à 13, caractérisé en ce qu'on ajoute à la laque durcissable par exposition à un rayonnement ultraviolet, un diluant ou un solvant supplémentaire à concurrence de moins de 6% en poids; de préférence, on n'ajoute aucun diluant et/ou aucun solvant.

15. Procédé selon une ou plusieurs des revendications 1 à 14, caractérisé en ce qu'on fait fonctionner la ou les premières sources de rayonnements sous l'atmosphère d'un gaz inerte, de préférence sous atmosphère d'azote.

16. Procédé selon une ou plusieurs des revendications 1 à 15, caractérisé en ce qu'on utilise des prépolymères qui ne contiennent pas de diluants (et qui ne contiennent pas d'acrylates monomères, c'est-à-dire des prépolymères à 100%) et on met en oeuvre des prépolymères possédant une viscosité de 0,1 à 200 Pa.s à 23°C, de préférence de 0,15 à 50 Pa.s.

17. Procédé selon une ou plusieurs des revendications 1 à 16, caractérisé en ce que le ou les émetteurs de rayonnement ultraviolet ultérieurs (possédant une longueur d'onde de 202 à 900 nm) présente, respectivement présentent une puissance et/ou une intensité de rayonnement supérieures à celles du, respectivement des premiers émetteurs de rayonnement ultraviolet.

18. Procédé selon une ou plusieurs des revendications 1 à 17, caractérisé en ce qu'on dispose, après le, respectivement les premiers émetteurs de rayonnement dont le spectre ultraviolet s'étend de 100 à 200 nm, de préférence après' un émetteur de rayonnement monochromatique de 172 nm, un émetteur de rayonnement électronique qui remplace le, respectivement les émetteurs de rayonnement ultraviolet dont la gamme de longueurs d'ondes est supérieure à 200 nm.
